(19) Europäisches Patentamt
European Patent Office
Office européen des brevets

(11) Publication number: **0 043 980**
**B1**

(12) **EUROPEAN PATENT SPECIFICATION**

(45) Date of publication of patent specification: **16.09.87**

(51) Int. Cl.⁴: **C 07 K 1/00,** C 07 H 21/04, C 12 N 15/00, C 12 P 21/02, A 61 K 45/02

(21) Application number: **81105067.3**

(22) Date of filing: **30.06.81**

(54) **Mature human leukocyte interferon A, process for its microbial preparation, intermediates therefor and compositions containing it.**

(30) Priority: **01.07.80 US 164986**
**08.09.80 US 184909**
**10.11.80 US 205578**
**21.04.81 US 256204**

(43) Date of publication of application:
**20.01.82 Bulletin 82/03**

(45) Publication of the grant of the patent:
**16.09.87 Bulletin 87/38**

(84) Designated Contracting States:
**AT BE DE NL SE**

(56) References cited:
**EP-A-0 032 134**
**EP-A-0 034 307**
**EP-A-0 042 246**
**EP-A-0 051 873**
**FR-A-2 442 054**

**VIROLOGY, vol. 97, 1979, Academic Press, Inc. W.E. STEWART et al.: "Effect of glycosylation inhibitors on the production and properties of human leucocyte interferon" pages 473-476**

**NATURE, vol. 284, 27th March 1980 Macmillan Journals Ltd. S. NAGATA et al.: "Synthesis in E. coli of a polypeptide with human leukocyte interferon activity" pages 316-320**

(73) Proprietor: **F. HOFFMANN-LA ROCHE & CO. Aktiengesellschaft**
**CH-4002 Basel (CH)**

(73) Proprietor: **GENENTECH, INC.**
**460 Point San Bruno Boulevard**
**South San Francisco California 94080 (US)**

(72) Inventor: **Goeddel, David Van Norman**
**1449 Benito Avenue**
**Burlingame, California (US)**
Inventor: **Pestka, Sidney**
**82 Brookside Terrace**
**North Caldwell, New Jersey (US)**

(74) Representative: **Lederer, Franz, Dr. et al**
**Vanderwerth, Lederer & Riederer Patentanwälte**
**Lucile-Grahn-Strasse 22**
**D-8000 München 80 (DE)**

(56) References cited:
**NATURE, vol. 287, 2nd October 1980 no. 5781, Macmillan Journals Ltd. CHESHAM, BUCKS, (GB) D.V. GOEDDEL et al.: "Human leukocyte interferon produced by E. Coli is biologically active" pages 411-416**

**NUCLEIC ACIDS RESEARCH, vol. 9, no. 3, 1981, IRL Press LTD. LONDON (GB) E. YELVERTON et al.: "Bacterial synthesis of a novel human leukocyte interferon" pages 731-741**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 043 980 B1**

(56) References cited:

**SCIENCE, vol. 209, 19th September 1980 M. STREULI et al.: "At least three human type alpha interferons: structure of alpha 2" pages 1343-1347**

**GENE, vol. 10, May 1980 Elsevier/North-Holland Biomedical Press (NL) N. MANTEI et al.: "The nucleotide sequence of a cloned human leukocyte interferon cDNA" pages 1-10**

**NATURE, vol. 290, no. 5801, 5th March 1981 CHESHA M, BUCKS (GB) D.V. GOEDDEL et al. "The structure of eight distinct cloned human leukocyte interferon cDNAs" pages 20-26**

**Description**

The present invention relates to the field of recombinant DNA technology, i.e. to processes used in recombinant DNA technology and to products obtained by these processes.

In a more detailed aspect the present invention relates to mature human leukocyte interferon-A, to pharmaceutical compositions containing it and to a process for its preparation which comprises causing a culture of a microorganism transformed with a replicable microbial expression vehicle capable of expressing said polypeptide to grow up and express said polypeptide. The present invention also comprises the expression vehicles used in this process and the novel microorganisms containing these expression vehicles as well as the processes for the preparation. Finally, the invention relates to DNA sequences comprising sequences coding for the amino acid sequence of a mature human leukocyte interferon-A.

Background of the invention

Human leukocyte interferon (LeIF) was first discovered and prepared in the form of very crude precipitates by Isaacs and Lindenmann (Proc. R. Soc. B 147, 258—267 [1957]; U.S.P. 3.699.222). Efforts to purify and characterize the material have been ongoing since that time, and have led to the preparation of relatively homogeneous leukocyte interferons derived from normal or leukemic donors' leukocytes (German Offenlegungsschrift No. 2.947.134). These interferons are a family of proteins known to possess the ability to confer a virus-resistant state in their target cells. In addition, interferon can act to inhibit cell proliferation and modulate immune response. These properties have prompted the clinical use of leukocyte interferon as a therapeutic agent for the treatment of viral infections and malignancies.

Leukocyte interferons have been purified to essential homogeneity (Rubinstein et al., Proc. Natl. Acad. Sci. U.S.A. *76*, 640—644 [1979]; Zoon et al., ibid. *76*, 5601—5605 [1979]), and reported molecular weights range from about 17,500 to about 21,000. The specific activity of these preparations is remarkably high, $2\times10^8$ to $1\times10^9$ units/mg protein, but yields from cell culture methods have been discouragingly low. Nevertheless, advances in protein sequencing techniques have permitted the determination of partial amino acid sequences (Zoon et al., Science *207*, 527 [1980]; Levy et al., Proc. Natl. Acad. Sci. U.S.A. *77*, 5102—5104 [1980]). Elucidation of the glycosylation of various leukocyte interferons is not at present complete, but it is now clear that differences in glycosylation among family members does not alone account for the spectrum of molecular weights observed. Instead, the leukocyte interferons differ markedly in amino acid composition and sequence, and amino acid homology is, in some cases, less than 80 percent.

While isolation from donor leukocytes has provided sufficient material for partial characterization and limited clinical studies with homogeneous leukocyte interferon, it is a totally inadequate source for the amounts of interferon needed for large scale clinical trials and for broad scale prophylactic and/or therapeutic use thereafter. Indeed, presently clinical investigations employing human leukocyte-derived interferons in antitumor and antiviral testing have principally been confined to crude ($<1$ percent pure) preparations of the material, and long lead times for the manufacture of sufficient quantities, even at unrealistic price levels, have critically delayed investigation on an expanded front.

With the advent of recombinant DNA technology, however, the controlled microbial production of an enormous variety of useful polypepties has become possible. Already in hand are bacteria modified by this technology to permit the production of such polypeptide products such as somatostatin, the A and B chains of human insulin and human growth hormone (Itakura et al., Science 198, 1056—1063 [1977]; Goeddel et al., Nature *281*, 544—548 [1979]). More recently, recombinant DNA techniques have been used to occasion the bacterial production of proinsulin and thymosin alpha 1 and several authors have reported on the obtention of DNA coding for human leukocyte interferon and to resultant proteins having leukocyte interferon activity (Nagata et al., Nature *284*, 316—320 [1980]; Mantei et al., Gene *10*, 1—10 [1980]; Taniguchi et al., Nature *285*, 547—549 [1980]).

The workhorse of recombinant DNA technology is the plasmid, a non-chromosomal loop of double-stranded DNA found in bacteria and other microbes, oftentimes in multiple copies per cell. Included in the information encoded in the plasmid DNA is that required to reproduce the plasmid in daughter cells (i.e., a "replicon") and ordinarily, one or more selection characteristics such as, in the case of bacteria, resistance to antibiotics which permit clones of the host cell containing the plasmid of interest to be recognized and preferentially grown in selective media. The utility of plasmids lies in the fact that they can be specifically cleaved by one or another restriction endonuclease or "restriction enzyme", each of which recognizes a different site on the plasmidic DNA. Thereafter heterologous genes or gene fragments may be inserted into the plasmid by endwise joining at the cleavage site or at reconstructed ends adjacent to the cleavage site. DNA recombination is performed outside the cell, but the resulting "recombinant" plasmid can be introduced into it by a process known as transformation and large quantities of the heterologous gene-containing recombinant plasmid are obtained by growing the transformant. Moreover, where the gene is properly inserted with reference to portions of the plasmid which govern the transcription and translation of the encoded DNA message, the resulting expression vehicle can be used to actually produce the polypeptide sequence for which the inserted gene codes, a process referred to as expression.

Expression is initiated in a region known as the promoter which is recognized by and bound by RNA polymerase. In some cases, as in the tryptophan or "trp" promoter preferred in the practice of the present

invention, promotor regions are overlapped by "operator" regions to form a combined promotor-operator. Operators are DNA sequences which are recognized by so-called repressor proteins which serve to regulate the frequency of transcription initiation at a particular promotor. The polymerase travels along the DNA, transcribing the information contained in the coding strans from its 5′ to 3′ end into messenger RNA which is in turn translated into a polypeptide having the amino acid sequence for which the DNA codes. Each amino acid is encoded by a nucleotide triplet or "codon" within what may for present purposes be referred to as the "structural gene", i.e. that part which encodes the amino acid sequence of the expressed product. After binding to the promotor, the RNA polymerase first transcribes nucleotides encoding a ribosome binding site, then a translation initiation or "start" signal (ordinarily ATG, which in the resulting messenger RNA becomes AUG), then the nucleotide codons within the structural gene itself. So-called stop codons are transcribed at the end of the structural gene whereafter the polymerase may form an additional sequence of messenger RNA which, because of the presence of the stop signal, will remain untranslated by the ribosomes. Ribosomes bind to the binding site provided on the messenger RNA, in bacteria ordinarily as the mRNA is being formed, and themselves produce the encoded polypeptide, beginning at the translation start signal and ending at the previously mentioned stop signal. The desired product is produced if the sequences encoding the ribosome binding sites are positioned properly with respect to the AUG initiation codon and if all remaining codons follow the initiation codon in phase. The resulting product may be obtained by lysing the host cell and recovering the product by appropriate purification from other bacterial protein.

We perceived that application of recombinant DNA technology (i.e. the insertion of interferon genes in microbial expression vehicles and their expression under the control of microbial gene regulatory elements) would be the most effective way of providing large quantities of leukocyte interferon which, despite the absence in material so produced of the glycosylation characteristic of human-derived material, could be employed clinically in the treatment of a wide range of viral and neoplastic diseases.

The approach to obtaining a first leukocyte gene in accordance with the present invention involved the following steps:

(1) Partial amino acid sequences of human leukocyte interferon purified to homogeneity were used to construct sets of synthetic DNA probes the codons of which, in the aggregate, represented all possible nucleotide combinations capable of encoding the partial amino acid sequences.

(2) Bacterial colony banks were prepared containing complementary DNA (cDNA) from induced messenger RNA. Other induced mRNA having been radio-labelled was hybridized to plasmid cDNA from this bank. Hybridizing mRNA was eluted and tested for translation into interferon in oocyte assay. Plasmid DNA from colonies shown to induce interferon activity in this manner have further been tested for hybridization to probes made as described in (1) above.

(3) Parallel to the approach in part (2) above, induced mRNA-derived cDNA in plasmids were used to form an independent bank of transformant colonies. The probes of part (1) were used to prime the synthesis of radio-labelled single stranded cDNA for use as hybridization probes. The synthetic probes hybridized with induced mRNA as template and were extended by reverse transcription to form induced, radio-labelled cDNA. Clones from the colony bank that hybridized to radio-labelled cDNA obtained in this manner have been investigated further to confirm the presence of a full-length interfereon encoding gene. Any partial length putative gene fragment obtained in parts (1) or (2) can itself be used as a probe for the full-length gene.

(4) The full-length gene obtained above was tailored, using synthetic DNA, to eliminate any leader sequence that might prevent microbial expression of the mature polypeptide and to permit appropriate positioning in an expression vehicle relative to start signals and the ribosome binding site of a microbial promotor. Expressed interferon was purified to a point permitting confirmation of its character and determination of its activity.

(5) The interferon gene fragment prepared in the foregoing fashion was itself used in probing, by hybridization, for other partially homologous leukocyte interferon species.

In applying methods of recombinant DNA technology as outlined above the microbial production in high yield and purity of the family of homologous leukocyte interferons (unglysosylated) as mature polypeptides, essentially unaccompanied by the corresponding presequence or any portion thereof, was achieved. These interferons may be directly expressed, recovered and purified to levels fitting them for use in the treatment of viral and malignant diseases of animals and man. Family members so far expressed have proven efficacious in in vitro testing and, in the first such demonstration of its kind, in in vivo testing as well, the latter involving the first mature leukocyte interferon to have been microbially produced.

The expression "mature leukocyte interferon-A" used in the context of the present application defines a microbially (e.g. bacterially) produced interferon molecule, devoid of glycosyl groups and characterized by the following amino acid sequence:

Cys Asp Leu Pro Gln Thr His Ser Leu Gly Ser Arg Arg Thr Leu Met Leu Leu Ala Gln Met Arg Lys Ile Ser Leu Phe Ser Cys Leu Lys Asp Arg His Asp Phe Gly Phe Pro Gln Glu Glu Phe Gly Asn Gln Phe Gln Lys Ala Glu Thr Ile Pro Val Leu His Glu Met Ile Gln Gln Ile Phe Asn Leu Phe Ser Thr Lys Asp Ser Ser Ala Ala Trp Asp Glu Thr Leu Leu Asp Lys Phe Tyr Thr Glu Leu Tyr Gln Gln Leu Asn Asp Leu Glu Ala Cys Val Ile Gln Gly Val Gly Val Thr Glu Thr Pro Leu Met Lys Glu Asp Ser Ile Leu Ala Val Arg Lys Tyr Phe Gln Arg Ile Thr Leu Tyr Leu Lys

Glu Lys Lys Tyr Ser Pro Cys Ala Trp Glu Val Val Arg Ala Glu Ile Met Arg Ser Phe Ser Leu Ser Thr Asn Leu Gln Glu Ser Leu Arg Ser Lys Glu.

Mature leukocyte interferon-A according to the present invention, is immediately expressed from a translation start signal (ATG) just before the first amino acid codon of the natural product. The "mature" polypeptide may thus contain as the first amino acid in its sequence methionine (for which ATG codes) without essentially altering its character. On the other hand the microbial host may process the translation product to delete the initial methionine. Mature leukocyte interferon-A could be expressed together with a conjugated protein other than the conventional leader, the conjugate being specifically cleavable in an intra- or extracellular environment (see British Patent Publication No. 2007676A). Finally, the mature leukocyte interferon-A could be produced in conjunction with a microbial "signal" peptide which transports the conjugate to the cell wall, where the signal is processed away and the mature polypeptide secreted. "Expression" of mature leukocyte interferon-A connotes the bacterial or other microbial production of an interferon molecule containing no glycosyl groups or a presequence that immediately attends mRNA translation of a human leukocyte interferone genome.

The particular leukocyte interferon-A protein has been defined by means of determined DNA gene (Figure 3) and deductive amino acid sequencing (Figure 4). It will be understood that for this particular interferon, natural allelic variations exist and occur from individual to individual. These variations may be demonstrated by (an) amino acid difference(s) in the overall sequence or by deletions, substitutions, insertions, inversions or additions of (an) amino acid(s) in said sequence. For the leukocyte interferon-A protein hereof, labelled LeIF A, such allelic variations are included within the scope of the label or term defining such, and thus, this invention.

Description of the drawings

Figure 1 depicts two amino acid sequences which were common to all interferon species isolated from human leukocyte and purified to homogeneity designated T-1 and T-13. All potential mRNA sequences coding for these peptides are shown, as are the corresponding DNA sequences. The letters A, T, G, C and U respectively connote the nucleotides containing the bases adenine, thymine, guanine, cytosine and uracil. The letter N connotes any one of the nucleotides A, G, C and U. Polynucleotides are depicted as reading from the 5' (left) in the 3' (right) direction and, where double stranded ("d.s.") DNA is depicted, vice-versa for the bottom or non-coding strand.

Figure 2 is an autoradiogram showing hybridization of potential LeIF plasmids with $^{32}P$-labelled synthetic deoxyoligonucleotides.

Figure 3 depicts the nucleotide sequence (coding strand) of the gene fragment isolated as candidate for use in the expression of leukocyte interferon-A. The ATG translational initiation codon and the termination triplet is underlined. The stop codon or termination triplet is followed by a 3' untranslated region. A 5' untranslated region precedes the leader sequence. The full-length gene for LeIF A is missing one codon found in other leukocyte interferons, as indicated in the third line of Figure 3.

Figure 4 shows the LeIF A protein sequence predicted from nucleotide sequence. The one letter abbreviations recommended by the IUPAC-IUB Commission on Biochemical Nomenclature are used: A, alanine; C, cysteine; D, aspartic acid; E, glutamic acid; F, phenylalanine; G, glycine; H, histidine; I, isoleucine; K, lysine; L, leucine; M, methionine; N, asparagine; P, proline; Q, glutamine; R, arginine; S, serine; T, threonine; V, valine; W, tryptophan; and Y, tyrosine. The numbers refer to amino acid positions (S refers to signal peptide). The dash in the 165 amino acid LeIF A sequence at position 44 is introduced to align the LeIF A sequence with the 166 amino acid sequences of other LeIFs.

Figure 5 depicts the restriction endonuclease map of the LeIF A cloned cDNA. The hybrid plasmid was constructed by the dC:dG tailing method (Goeddel, D. V. et al., Nature *287*, 411—416 [1980]). Therefore, the cDNA insert can be excised using PstI. The lines at the end of the cDNA insert represent the flanking homopolymeric dC:dG tails. The positions of PvuII and BglII restriction sites are indicated. The shaded region of the figure represents the coding sequence of mature LeIF A; the cross-hatched region indicates the signal peptide coding sequence; and the open regions show 3' and 5' non-coding sequences.

Figure 6 schematically depicts the construction of a gene coding for the direct microbial synthesis of mature LeIF A. Restriction sites and residues are as shown ("Pst I", etc.). The term "b.p." connotes "base pair".

A. Microorganisms employed

The work described involved use of two microorganisms: E. coli×1776, as described in U.S.P. 4.190.495, and E. coli K-12 strain 294 (end A, thi$^-$, hsr$^-$, hsm$_k^+$), as described in British Patent Publication No. 2055382 A. Each has been deposited with the American Type Culture Collection (ATCC accession Nos. 31537 and 31446 respectively). All recombinant DNA work was performed in compliance with applicable guidelines of the National Institutes of Health.

The invention is described with reference to E. coli, including not only strains E. coli×1776 and E. coli K-12 strain 294, defined above, but also other known E. coli strains such as E. coli B.

B. Source and purification of LeIF mRNA

LeIF mRNA may be obtained from human leukocytes, ordinarily those of patients with chronic

myelogenous leukemia, that have been inducted to produce interferon with Sendai or Newcastle disease virus, as described in e.g. German Offenlegungsschrift No. 2947134. A particularly preferred source, and that used in the work reported herein, is a cell line designated KG-1 derived from a patient with acute myelogenous leukemia. The cell line, described by Koeffler, H. P. and Golde, D. W., Science *200*, 1153 (1978), grows readily in a culture medium comprising RPMI (Rosewell Park Memorial Institute) 1640 plus 10% FCS (fetal calf serum) heat-inactivated, 25 mM HEPES buffer (N-2-hydroxy-ethyl-piperazine-N'-2-ethane-sulfonic acid) and 50 µg/ml of gentamicin, and is subcultured 1 to 3 split two times a week. Cells may be frozen from the foregoing growth medium plus 10% dimethylsulfoxide. KG-1 has been deposited with the American Type Culture Collection (ATCC accession No. CRL 8031).

KG-1 cells were induced to produce leukocyte interferon mRNA with Sendai or Newcastle disease virus following the procedure described by Rubinstein et al. (Proc. Natl. Acad. Sci. U.S.A. *76*, 640—644 [1979]). Cells were harvested 5 hours after induction and RNA prepared by the guanidine thiocyanate-quanidine hydrochloride procedure (Chirgwin et al., Biochemistry *18*, 5294—5299 [1979]). RNA from uninduced cells was isolated in the same manner. Oligo deoxythymidin (dT)—cellulose chromatography and sucrose gradient ultracentrifugation was used to obtain the 12S fraction of poly (A) mRNA as described by Green et al., (Arch. Biochem. Biophys. *172*, 74—89 [1976]) and Okuyuma et al. (Arch. Biochem. Biphys. *188*, 98—104 [1978]). This mRNA had an interferon titer of 8000—10,000 units per microgram in the Xenopus laevis oocyte assay (Cavalieri et al., Proc. Natl. Acad. Sci. U.S.A. *74*, 3287—3291 [1977]).

C. Preparation of colony banks containing LeIF cDNA sequences

5 µm of mRNA was used to prepare double stranded cDNA by standard procedures (Wickens et al., J. Biol. Chem. *253*, 2483—2495 [1978] and Goeddel et al., Nature *281*, 544—548 [1979]). The cDNA was size fractionated by electrophoresis on a 6% polyacrylamide gel and 230 ng of material ranging in size from 500 to 1500 b.p. were recovered by electroelution. A 100 ng portion of this cDNA was tailed with deoxycytidin (dC) residues as described by Chang et al., Nature *275*, 617—624 (1978), annealed with 470 ng of plasmid pBR322 which has been tailed with deoxyguanosin (dG) residues at the PstI site (Bolivar et al., Gene *2*, 95—113 [1977]), and used to transform E. coli×1776. Approximately 130 tetracycline resistant, ampicillin sensitive transformants were obtained per ng of cDNA.

In a second similar experiment, approximately 1000 tetracycline resistant, ampicillin sensitive E. coli K-12 strain 294 transformants were obtained per ng of cDNA. In this case size fractionated cDNA material ranging in size from 600 to 1300 b.p. was recovered by electroelution for dC tailing.

D. Preparation of synthetic oligonucleotides and their use

The knowledge of the amino acid sequences of several tryptic fragments of human leukocyte interferon permitted the design of synthetic deoxyoligonucleotides complementary to different regions of LeIF mRNA. The two tryptic peptides T1 and T13 were selected because they had amino acid sequences requiring the synthesis of only 12 and 4 undecamers, respectively, to account for all possible coding sequences (Figure 1.). Four sets of deoxyoligonucelotide probes were synthesized for each sequence, containing either three (T-1A, B, C, D) or one (T-13A, B, C, D) oligonucleotide each. The indicated complementary deoxyoligonucleotides 11 bases long were chemically synthesized by the phosphotriester method (Crea et al., Proc. Natl. Acad. Sci. U.S.A. *75*, 5765—5769 [1978]). Four individual probes were prepared in the T-13 series. The twelve T-1 probes were prepared in four pools of three probes as shown in Figure 1.

The four individual probes of the T-13 series and the twelve T-1 probes prepared in four pools of three primers each were used to prime the synthesis of radiolabelled single stranded cDNA for use as hybridization probes. The template mRNA was either the 12S RNA from Sendai-induced KG-1 cells (8000 units IF activity per µg) or total poly (A) mRNA from uninduced leukocytes (<10 units per µg). $^{32}$P-labelled cDNA was prepared from these primers using known reaction conditions (Noyes et al., Proc. Natl. Acad. Sci. U.S.A. *76*, 1770—1774 [1979]). The 60 µl reactions were performed in 20 mM Tris-HCl (pH 8.3), 20 mM KCl, 8 mM $MgCl_2$, 30 mM β-mercaptoethanol. Reactions included one µg of each primer (i.e. 12 µg total for T-1 series, 4 µm total for T-13 series), 2 µg of "induced" 12S fraction mRNA (or 10 µg of uninduced poly (A) mRNA), 0.5mM dATP, dCTP, dTTP, 200 µCi ($\alpha^{32}$ P)dCTP (Amersham, 2—3000 Ci/mmole), and 60 units reverse transcriptase (Bethesda Research Laboratories). Product was separated from unincorporated label by gel filtration on a 10 ml Sephadex® G-50 column, treated with 0.3N NaOH for 30 minutes at 70°C to destroy RNA, and neutralized with HCl. Hybridizations were performed as described by Kafatos et al., Nucleic Acids Res. *7*, 1541—1552 (1979).

E. Identification of clones pL1—pL30

The rapid plasmid isolation procedure of Birnboim et al., Nucleic Acids Res. *7*, 1513—1523 (1979) was used to prepare 1 µg of plasmid DNA from each of 500 individual E. coli K-12 strain 294 transformants (see C). Each DNA sample was denatured and applied to nitrocellulose filters in triplicate following the procedure of Kafatos et al. (see above).

The three sets of nitrocellulose filters containing the 500 plasmid samples were hybridized with

a) induced cDNA primed with the T-1 set of primers.

b) T-13 primed induced cDNA, and

c) uninduced cDNA prepared by using both sets of primers. Clones were considered positive if they hybridized more strongly to one or both of the induced cDNA probes than to the total uninduced probe. Thirty "positive" clones (pL1—pL30) were selected from the 500 for further analysis.

F. Identification of clones pL31—pL39. Isolation of a plasmid (No. 104) containing a LeIF gene fragment.

Transformants of E. coli×1776 were screened by the colony hybridization procedure of Grunstein and Hogness (Proc. Natl. Acad. Sci. U.S.A. *72*, 3961—3965 [1975]) using $^{32}P$-labelled induced mRNA as probe (Lillenhaug et al., Biochemistry, *15*, 1858—1865 [1976]). Unlabelled mRNA from uninduced cells was mixed with the probe at a ratio of 200 to 1 to compete with uninduced mRNA present in the $^{32}P$-labelled preparation. Hybridization of labelled mRNA should occur preferentially to colonies containing induced sequences. Three classes of transformants were obtained:

(1) 2—3% of the colonies hybridized to $^{32}P$-mRNA very strongly,

(2) 10% hybridized significantly less than class 1, and

(3) the remainder gave no detectable hybridization signal.

The positive colonies (classes (1) and (2)) were examined for the presence of interferon-specific sequences by an assay which depends upon hybridization of interferon mRNA specifically to plasmid DNA. Initially, 60 strong positive colonies (class 1) were grown individually in 100 ml of M9 medium supplemented with tetracycline (20 µg/ml), diaminopimelic acid (100 µg/ml), thymidine (20 µg/ml), and d-biotin (1 µg/ml). The M9 medium contains per liter $Na_2HPO_4$ (6g), $KH_2PO_4$ (3 g), NaCl (0.5 g) and $NH_4Cl$ (1 g). After autoclaving 1 ml of sterile 1M $MgSO_4$ and 10 ml of sterile 0.01 M $CaCl_2$ are added. Ten cultures were pooled and plasmid DNA was isolated from the six pools as described by Clewell et al., Biochemistry *9*, 4428—440 [1970]. Ten µg of each plasmid DNA pool were cleaved with HindIII, denatured and covalently bound to DBM (diazobenzyloxymethyl) paper. One µg of purified mRNA from induced cells was hybridized to each filter. Unhybridized mRNA was removed by washing. The specifically hybridized mRNA was eluted and translated in Xenopus laevis oocytes. By this assay, all six pools were negative. Five pools of ten colonies each and one pool of nine colonies were made from 59 weakly positive colonies (class 2) and plasmids were prepared from the pools and examined as above. Among the six pools tested, one (K10) hybridized to interferon mRNA at levels significantly above background levels each time it was tested. In order to identify the specific interferon cDNA clone plasmid DNAs were prepared from the 9 colonies of pool K10 and examined individually. Two of the nine plasmids (No. 101 and No. 104 bound interferon mRNA well above background levels. From plasmid No. 104 a unique Bgl II restriction fragment containing 260 b.p. was isolated, labelled with $^{32}P$ using the procedure described by Taylor et al., Biochim, Biophys. Acta *442*, 324—330 (1976), and used as probe to independently screen 400 E. coli 294 transformants by an in situ colony screening procedure (Grunstein and Hogness, Proc. Natl. Sci. U.S.A. *72*, 3961—3965 [1975]). Nine colonies (pL31—pL39) were identifed which hybridized to different extents with this probe.

In addition, the labelled 260 b.p. fragment was used to indpendently screen 4000 E. coli 294 transformants in the same manner. 50 colonies were identified which hybridized to different extents with this probe.

G. Isolation and sequencing of a first full-length LeIF gene

Plasmid DNA was prepared from all 39 potential LeIF cDNA clones and rescreened with the same 260 b.p. DNA probe using the hybridization procedure of Kafatos et al., (see above). Three plasmids (pL4, pL31, pL34) gave very strong hybridization signals, four (pL13, pL30, pL32, pL36) hybridized moderately, and three (pL6, pL8, pL14) hybridized weakly with the probe.

The 39 potential LeIF cDNA recombinant plasmids were also screened by using $^{32}P$-labelled synthetic undercamers (individual T-1 primer pools or individual T-13 primers) directly as hybridization probes. The hybridization conditions were chosen such that perfect base pairing should be required for detectable hybridization signals (Wallace et al., Nucleic Acids Res. *6* 3543—3557 [1979]). Thus, plasmid DNA from the 39 clones was prepared by a standard cleared lysate procedure (Clewell et al., see above) and purified by Biorad Agarose A-50 column chromatography Samples (3 µg) of each preparation were linearized by treatment with Eco RI, denatured in alkali and spotted on 2 separate nitrocellulose filters, 1.5 µg per spot (Kafatos et al., see above). Individual synthetic deoxyoligonucleotide primers and primer pools were phosphorylated with ($\gamma^{32}$ P)ATP as follows: 50 pmoles of oligonucleotide and 100 pmoles of ($\gamma^{32}P$)ATP (New England Nuclear, 2500 Ci/mmole) were combined in 30 µl of 50 mM Tris-HCl, 10 mM $MgCl_2$ 15 mM β-mercaptoethanol. 2 units of T4 polynucleotide kinase were added and, after 30 minutes at 37°C, $^{32}P$-labelled primers were purified by chromatography on 10 ml Sephadex® G-50 columns. Hybridizations were performed using $10^6$ cpm or primer T-13C or $3\times10^6$ cpm of primer pool T-1C. The hybridizations were performed at 15°C for 14 hours in 6×SSC [1×SSC=0.15 M NaCl, 0.015 M sodium citrate, pH 7.2], 10×Denhardt's [0.2% bovine serum albumine, 0.2% polyvinylpyrolidone, 0.2% Ficoll] solution, as described by Wallace et al. (see above). Filters were washed for 5 minutes (3 times) at 0°C in 6×SSC, dried, and exposed to x-ray film. Results are shown in Fig. 2 for $^{32}P$-primer pool T-13C and primer T-1C.

Plasmid DNA from clone 104 was found to give significant hybridization with primer pool T-1C and primer T-13C, but no detectable hybridization with the other undecamers. As shown in Figure 2, several of the 39 potential LeIF plasmids (pL2, 4, 13, 17, 20, 30, 31, 34) also hybridized with both of these probes. However, restriction analysis showed that only one of these plasmids, pL31, also contained a 260 b.p.

internal BglII fragment. PstI digestion of pL31 showed the size of the cDNA insert to be approximately 1000 b.p.

The entire PstI insert of pL31 was sequenced by both the Maxam-Gilbert chemical method (Methods Enzymol. *65*, 499—560 [1980]) and by the dideoxy chain termination procedure (Smith, Methods Enzymol. *65*, 560—580 [1980]) after subcloning Sau3a fragments into an M13 vector.

The DNA sequence is shown ("A") in Figure 3. The appropriate translational reading frame could be predicted from protein sequence information in hand, the known range of LeIF molecular weights, and the relative incidence of stop triplets in the three possible reading frames, and that in turn permitted prediction of the entire LeIF amino acid sequence, including a pre- or signal peptide. The first ATG translational initiation codon is found 60 nucleotides from the 5′ end of the sequence and is followed, 188 codons later, by a TGA termination triplet; there are 342 untranslated nucleotides at the 3′ end, followed by a poly (A) sequence. The putative signal peptide (presumably involved in the secretion of mature LeIF from leukocytes) is 23 amino acids long. The 165 amino acids constituting the mature LeIF have a calculated MW of 19,390. We have termed the LeIF encoded by pL31 "LeIF A."

H. Direct expression of mature leukocyte interferon A (LeIF A)

1. Generally

The procedure followed by express LeIF A directly as a mature interferon polypeptide is a variant of that earlier employed for human growth hormone (Goeddel et al., Nature *281*, 544—548 [1979]), insofar as it involved the combination of synthetic (N-terminal) and complementary DNAs.

As shown in Figure 6, a Sau3a restriction endonuclease site is conveniently located between codons 1 and 3 of LeIF A. Two synthetic deoxyoligonucleotides were designed which incorporate an ATG translational initiation codon, restore the codon for amino acid 1 (cysteine), and create an EcoRI sticky end. These oligomers were ligated to a 34 b.p. Sau3a—AvaII fragment of pL31. The resulting 45 b.p. product was ligated to two additional DNA fragments to construct an 865 b.p. synthetic-natural hybrid gene which codes for LeIF A and which is bounded by EcoRI and PstI restriction sites. This gene was inserted into pBR322 between the EcoRI and PstI sites to give the plasmid pLeIF A1.

2. Construction of the tryptophan control element (containing the E. coli trp promoter, operator and trp leader ribosome binding site but lacking an ATG sequence for initiation of translation).

Plasmid pGMI carries the E. coli tryptophan operon containing the deletion ΔLE1413 (Miozzari et al., J. Bacteriology *133*, 1457—1466 [1978]) and hence expresses a fusion protein comprising the first 6 amino acids of the trp leader and approximately the last third of the trp E polypeptide (hereinafter referred to in conjunction as LE′), as well as the trp D polypeptide in its entirety, all under the control of the trp promoter-operator system. The plasmid, 20 μg, was digested with the restriction enzyme PvuII which cleaves the plasmid at five sites. The gene fragments were next combined with EcoRI linkers (consisting of a self complementary oligonucleotide of the sequence: pCATGAATTCATG) providing an EcoRI cleavage site for a later cloning into a plasmid containing an EcoRI site. The 20 μg of DNA fragments obtained from pGM1 were treated with 10 units $T_4$ DNA ligase in the presence of 200 pmoles of the 5′-phosphorylated synthetic oligonucleotide pCATGAATTCATG and in 20 μl $T_4$ DNA ligase buffer (20 mM Tris, pH 7.6, 0.5 mM ATP, 10 mM $MgCl_2$, 5 mM dithiothreitol) at 4°C overnight. The solution was then heated 10 minutes at 70°C to halt ligation. The linkers were cleaved by EcoRI digestion and the fragments, now with EcoRI ends were separated using 5 percent polyacrylamide gel electrophoresis (hereinafter PAGE) and the three largest fragments isolated from the gel by first staining with ethidium bromide, locating the fragments with ultraviolet light, and cutting from the gel the portions of interest. Each gel fragment, with 300 microliters 0.1×TBE, was placed in a dialysis bag and subjected to electrophoresis at 100 V for one hour in 0.1×TBE buffer (TBE buffer contains: 10.8 g Tris base, 5.5 g boric acid, 0.09 g $Na_2EDTA$ in 1 liter $H_2O$). The aqueous solution was collected from the dialysis bag, phenolextracted, chloroform extracted and made 0.2 M sodium chloride, and the DNA recovered in water after ethanol precipitation. The trp promoter-operator-containing gene with EcoRI sticky ends was identified in the procedure next described, which entails the insertion of fragments into a tetracycline sensitive plasmid which, upon promoter-operator insertion, becomes tetracycline resistant.

Plasmid pBRHI (Rodriguez et al., Nucleic Acids Res. *6*, 3267—3287 [1979]) expresses ampicillin resistance and contains the gene for tetracycline resistance but, there being no associated promoter, does not express that resistance. The plasmid is accordingly tetracycline sensitive. By introducing a promoter-operator system in the EcoRI site, the plasmid can be made tetracycline resistant.

pBRH1 was digested with EcoRI and the enzyme removed by phenol extraction followed by chloroform extraction and recovered in water after ethanol precipitation. The resulting DNA molecule was, in separate reaction mixtures, combined with each of the three DNA fragments obtained above and ligated with $T_4$ DNA ligase as previously described. The DNA present in the reaction mixture was used to transform competent E. coli K-12 strain 294 by standard techniques (Hershfield et al., Proc. Natl. Acad. Sci. U.S.A. *71*, 3455—3459 [1974]) and the bacteria plated on LB (Luria-Bertani) plates containing 20 μg/ml ampicillin and 5 μg/ml tetracycline. Several tetracycline-resistant colonies were selected, plasmid DNA isolated and the presence of the desired fragment confirmed by restriction enzyme analysis. The resulting plasmid is designated pBRHtrp.

An EcoRI and BamHI digestion product of the viral genome of hepatitis B was obtained by conventional means and cloned into the EcoRI and BamHI sites of plasmid pGH6 (Goeddel et al, Nature *281*, 544 [1979]) to form the plasmid pHS32. Plasmid pHS32 was cleaved with XbaI, phenol extracted, chloroform extracted and ethanol precipitated. It was then treated with 1 μl E. coli DNA polymerase I, Klenow fragment (Boehringer-Mannheim) in 30 μl polymerase buffer (50 mM potassium phosphate pH 7.4, 7 mM $MgCl_2$, 1 mM β-mercaptoethanol) containing 0.1 mM dTTP and 0.1 mM dCTP for 30 minutes at 0°C then 2 hours at 37°C. This treatment causes 2 of the 4 nucleotides complementary to the 5′ protruding end of the XbaI cleavage site to be filled in:

| | | | |
|---|---|---|---|
| 5′ | CTAGA— | 5′ | CTAGA— |
| 3′ | T— | 3′ | TCT— |

Two nucleotides, dC and dT, were incorporated giving and end with two 5′ protruding nucleotides. This linear residue of plasmid pHS32 (after phenol and chloroform extraction and recovery in water after ethanol precipitation) was cleaved with EcoRI. The large plasmid fragment was separated from the smaller EcoRI-XbaI fragment by PAGE and isolated after electroelution. This DNA fragment from pHS32 (0.2 μg), was ligated, under conditions similar to those described above, to the EcoRI—TaqI fragment of the tryptophan operon (~0.01 μg), derived from pBRHtrp.

In the process of ligating the fragment from pHS32 to the EcoRI—TaqI fragment, as described above, the TaqI protruding end is ligated to the XbaI remaining protruding end even though it is not completely Watson-Crick base-paired:

| | | | | |
|---|---|---|---|---|
| —T | | CTAGA— | | —TCTAGA— |
| | + | | → | |
| —AGC | | TCT— | | —AGCTCT— |

A portion of this ligation reaction mixture was transformed into E. coli 294 cells, heat treated and plated on LB plates containing ampicillin. Twenty-four colonies were selected, grown in 3 ml LB (Luria-Bertani) media, and plasmid isolated. Six of these were found to have the XbaI site regenerated via E. coli catalyzed DNA repair and replication:

| | | |
|---|---|---|
| —TCTAGA— | | —TCTAGA— |
| | → | |
| —AGCTCT— | | —AGATCT— |

These plasmids were also found to cleave both with EcoRI and HpaI and to give the expected restriction fragments. One plasmid, designated pTrp14, was used for expression of heterologous polypeptides, as next discussed.

The plasmid pHGH 107 (Goeddel et al., Nature *281*, 544, [1979]) contains a gene for human growth hormone made up of 23 amino acid codons produced from synthetic DNA fragments and 163 amino acid codons obtained from complementary DNA produced via reverse transcription of human growth hormone messenger RNA. This gene, though it lacks the codons of the "pre" sequence of human growth hormone, does contain an ATG translation initiation codon. The gene was isolated from 10 μg pHGH 107 after treatment with EcoRI followed by E. coli DNA polymerase I Klenow fragment and dTTP and dATP as described above. Following phenol and chloroform extraction and ethanol precipitation the plasmid was treated with BamHI.

The human growth hormone (HGH) gene-containing fragment was isolated by PAGE followed by electroelution. The resulting DNA fragment also contains the first 350 nucleotides of the tetracycline resistance structural gene, but lacks the tetracycline promoter-operator system so that, when subsequently cloned into an expression plasmid, plasmids containing the insert can be located by the restoration of tetracycline resistance. Because the EcoRI end of the fragment has been filled in by the Klenow polymerase I procedure, the fragment has one blunt and one sticky end, ensuring proper orientation when later inserted into an expression plasmid.

The expression plasmid pTrp14 was next prepared to receive the HGH gene-containing fragment prepared above. Thus, pTrp14 was XbaI digested and the resulting sticky ends filled in with the Klenow polymerase I procedure employing dATP, dTTP, dGTP and dCTP. After phenol and chloroform extraction and ethanol precipitation the resulting DNA was treated with BamHI and the resulting large plasmid fragment isolated by PAGE and electroelution. The pTrp14-derived fragment had one blunt and one sticky end, permitting recombination in proper orientation with the HGH gene containing fragment previously described.

The HGH gene fragment and the pTrp14 ΔXba-BamHI fragment were combined and ligated together under conditions similar to those described above. The filled in XbaI and EcoRI ends ligated together by blunt end ligation to recreate both the XbaI and the EcoRI site:

| Xbal filled in | | EcoRI filled in | | HGH gene initiation |
|---|---|---|---|---|
| —TCTAG | + | AATTCTATG— | → | —TCTAGAATTCTATG— |
| —AGATC | | TTAAGATAC— | | —AGATCTTAAGATAC— |
| | | | | Xbal EcoRI |

This construction also recreates the tetracycline resistance gene. Since the plasmid pHGH 107 expresses tetracycline resistance from a promoter lying upstream from the HGH gene (the lac promoter), this construction designated pHGH 207, permits expression of the gene for tetracycline resistance under the control of the tryptophan promoter-operator. Thus the ligation mixture was transformed into E. coli 294 and colonies selected on LB plates containing 5 μg/ml tetracycline.

Plasmid pHGH 207 was EcoRI digested and a 300 b.p. fragment containing the trp promoter, operator and trp leader ribosome binding site but lacking an ATG sequence for initiation of translation was recovered by PAGE followed by electroelution. This DNA fragment was cloned into the EcoRI site of pLeIF A. Expression plasmids containing the above modified trp regulon (E. coli trp operon from which the attenuator sequence has been deleted to controllably heighten expression levels) can be grown to predetermined levels in nutrient media containing additive tryptophan in quantities sufficient to repress the promoter-operator system, then be deprived of tryptophan so as to derepress the system and occasion the expression of the intended product.

More particularly, and with reference to Figure 6, 250 μg of plasmid pL31 were digested with PstI and and 1000 b.p. insert isolated by gel electrophoresis on a 6% polyacrylamide gel. Approximately 40 μg of insert was electroeluted from the gel and divided into 3 aliquots for further digestion: a) A 16 μg sample of this fragment was partially digested with 40 units of BglII for 45 minutes at 37°C and the reaction mixture purified on a 6% polyacrylamide gel. Approximately 2 μg of the desired 670 b.p. fragment were recovered. b) Another sample (8 μg) of the 1000 b.p. PstI insert was restricted with AvaII and BglII. One θg of the indicated 150 b.p. fragment was recovered after gel electrophoresis. c) 16 μg of the 1000 b.p. piece was treated with Sau3a and AvaII. After electrophoresis on a 10% polyacrylamide gel, approximately 0.25 μg (10 pmole) of the 34 b.p. fragment was recovered. The two indicated deoxyoligonucleotides, 5'-dAATTCATGTGT (fragment 1) and 5'-dGATCACACATG (fragment 2) were synthesized by the phosphotriester procedure. Fragment 2 was phosphorylated as follows. 200 μl (~40 pmole) of ($\gamma^{32}P$) ATP (Amersham, 5000 Ci/mmole) was dried down and resuspended in 30 μl of 60 mM Tris-HCl (pH8), 10 mM $MgCl_2$, 15 mM β-mercaptoethanol, containing 100 pmoles of DNA fragment and 2 units of T4 polynucleotide kinase. After 15 minutes at 37°C, 1 μl of 10 mM ATP was added and the reaction allowed to proceed another 15 minutes. The mixture was then heated at 70°C for 15 minutes, combined with 100 pmole of 5'-OH fragment 1 and 10 pmole of the 34 b.p. Sau3a—AvaII fragment. Ligation was performed for 5 hours at 4°C in 50 μl of 20 mM Tris-HCl (pH 7.5) 10mM Mg $Cl_2$, 10 mM dithiothreitol, 0.5 mM ATP and 10 units T4 DNA ligase. The mixture was electrophoresed on a 6% polyacrylamide gel and the 45 b.p. product recovered by electroelution. About 30 ng (1 pmole) of the 45 b.p. product were combined with 0.5 μg (5 pmoles) of the 150 b.p. AvaII—BglII fragment and 1 μg (2 pmoles) of the 670 b.p. BglII—PstI fragment. The ligation was performed at 20°C for 16 hours using 20 units of T4 DNA ligase. The ligase was inactivated by heating to 65°C for 10 minutes. The mixture was then digested with EcoRI and PstI to eliminate polymers of the gene. The mixture was purified by 6% PAGE. About 20 ng (0.04 pmole) of the 865 b.p. product were isolated. One-half (10ng) of this was ligated into pBR322 (0.3 μg) between the EcoRI and PstI sites. Transformation of E. coli 294 gave 70 tetracycline resistant, ampicillin sensitive transformants. Plasmid DNA isolated from 18 of these transformants were digested with EcoRI and PstI. 16 of the 18 plasmids had an EcoRI—PstI fragment 865 b.p. in length. One μg of one of these, pLeIF A1, was digested with EcoRI and ligated to the 300 b.p. EcoRI fragment (0.1 μg) containing the E. coli trp promoter and trp leader ribosome binding site, prepared as described above. Transformants containing the trp promoter were identified using a $^{32}P$-trp probe in conjunction with the Grunstein-Hogness colony screening procedure. An asymetrically located XbaI site in the trp fragment allowed determination of recombinants in which the trp promoter was oriented in the direction of the LeIF A gene.

I. In vitro and in vivo activity of LeIF A

Extracts were prepared for IF assay as follows: one ml cultures were grown in L broth containing 5 μg/ml tetracycline to an $A_{550}$ value of about 1.0, then diluted into 25 ml of M9 media containing 5 μg/ml tetracycline. 10 ml samples were harvested by centrifugation when $A_{550}$ reached 1.0 and cell pellets were suspended in 1 ml of 15 percent sucrose, 50 mM Tris-HCl (pH 8.0), 50 mM EDTA. One mg of lysozyme was added and, after 5 minutes at 0°C, cells were disrupted by sonication. The samples were centrifuged 10 minutes (15,000 rpm) and interferon activity in the supernatants was determined by comparison with LeIF standards by the cytopathic effect (CPE) inhibition assay. To determine the number of IF molecules per cell a LeIF specific activity of $4\times10^8$ units/mg was used.

As shown in Table 1, clone pLeIF A trp 25, in which the trp promoter was inserted in the desired orientation, gives high levels of activity (as high as $2.5\times10^8$ units per liter). As shown in Table 2, the IF

produced by E. coli K-12 strain 294/pLeIF A trp 25 behaves like authentic human LeIF; it is stable to treatment at pH 2 and is neutralized by rabbit anti-human leukocyte antibodies. The interferon has an apparent molecular weight of approximately 20,000.

The in vivo efficacy of interferon requires the presence of macrophages and natural killer (NK) cells and the in vivo mode of action appears to involve stimulation of these cells. Thus, it remained possible that the interferon produced by E. coli 294/pLeIF A 25, while having antiviral activity in the cell culture assay, would not be active in infected animals. Moreover, the in vivo antiviral activity of the bacterially produced, non-glycosylated LeIF A might be different from the glycosylated LeIF derived from human "buffy coat" leukocytes. Therefore the biological activity of bacterially synthesized LeIF A (2% pure) was compared with buffy coat LeIF (8% pure) in lethal encephalomyocarditis (EMC) virus infection of squirrel monkeys (Table 3).

TABLE 1
Interferon activity in extracts of E. coli

| E. coli K-12 strain 294 transformed by | Cell density (cells/ml) | IF Activity units/ml culture | LeIF molecules per cell |
|---|---|---|---|
| pLeIF A trp 25 | $3.5\times10^8$ | 36,000 | 9,000 |
| pLeIF A trp 25 | $1.8\times10^9$ | 250,000 | 12,000 |

TABLE 2
Comparison of activities of extracts from E. coli 294/pLeIF A 25 with standard LeIF*

| | Interferon activity (units/ml) | | |
|---|---|---|---|
| | Untreated | pH 2 | Rabbit anti-human leukocyte antibodies |
| 294/pLeIF A trp 25 extract | 500 | 500 | <10 |
| LeiF standard | 500 | 500 | <10 |

* The 250,000 units/ml extract of E. coli 294/pLeIF A trp 25 described in Table 1 was diluted 500-fold with minimal essential medium giving a specific activity of 500 units/ml. A leukocyte interferon standard (Wadley Institute) previously titrated against the NIH leukocyte interferon standard was also diluted to a final concentration of 500 U/ml. One ml aliquots were adjusted to pH 2 with 1N HCl, incubated at 4°C for 52 hours, neutralized by addition of NaOH and IF activity determined by the standard CPE inhibition assay. 25 µl aliquots of the 500 units/ml samples (untreated) were incubated with 25 µl of rabbit anti-human leukocyte interferon for 60 minutes at 37°C, centrifuged at 12,000×g for 5 minutes and the supernatant assayed.

TABLE 3

Antiviral effect of various LeIF preparations against EMC virus infection of squirrel monkeys

| Treatment | Survivors | Serum plaque forming units/ml Day 2 | Day 3 | Day 4 |
|---|---|---|---|---|
| Control | 0/3 | 10 | $3\times10^4$ | $10^5$ |
| (bacterial proteins) | | 0 } 3 | 0 } $10^4$ | 1,200 } $>3.4\times10^4$ |
| | | 0 | 0 | 0 |
| Bacterial LeIF A | 3/3 | 0 | 0 | 0 |
| | | 0 | 0 | 0 |
| | | 0 | 0 | 0 |
| LeIF standard | 3/3 | 0 | 0 | 0 |
| | | 0 | 0 | 0 |
| | | 0 | 0 | 0 |

All monkeys were male (average weight 713 g) and had no EMC virus antibodies prior to infection. The monkeys were infected intramuscularly with $100\times LD_{50}$ EMC virus (determined in mice). The control treated monkeys died at 134, 158 and 164 hours post infection. Interferon treatments with $10^6$ units were by the intravenous route at −4, +2, 23, 29, 48, 72, 168 and 240 hours, relative to infection. The bacterial leukocyte interferon was a column chromatography fraction from a lysate of E. coli 294/pLeIF A 25 at a specific activity of $7.4\times10^6$ units/mg protein. The control bacterial proteins were an equivalent column fraction from a lysate of E. coli 294/pBR322 at twice the total protein concentration. The leukocyte interferon standard was Sendai virus induced interferon from normal human "buffy-coat" cells, purified chromatographically to a specific activity of $32\times10^6$ units/mg protein.

The control monkeys showed progressive lethargy, loss of balance, flaccid paralysis of the hind-limbs and watering of the eyes commencing around 8 hours prior to death. The interferon treated monkeys showed none of these abnormalities; they remained active at all times and developed no viremia. The one monkey in the control group which did not develop viremia by 4 days died latest (164 hours post infection) but showed high titers of virus in the heart and brain on post mortem. The interferon treated monkeys did not develop antibodies to EMC virus as determined 14 and 21 days after infection. These results demonstrate that the antiviral effects of LeIF preparations in the infected animals can be attributed solely to interferon because the contaminating proteins are quite different in the bacterial and buffy coat preparations. In addition these results indicate that glycosylation is not required for the in vivo antiviral activity of LeIF A.

J. Purification

The content of mature leukocyte interferon A in bacterial extracts may be enhanced by successive:

1. Polyethylene—imine precipitation, in which most of the cellular protein, including the interferon, remains in the supernatant.

2. Ammonium sulfate fractionation in which interferon comes out of solution in 55% saturated ammonium sulfate.

3. Suspension of the ammonium sulfate pellet in 0.06 M potassium phosphate, 10 mM Tris-HCl, pH 7.2, and dialysis against 25 mM Tris-HCl, pH 7.9 (interferon activity remains in solution).

4. Chromatography of the above supernantant, pH adjusted to 8.5, on a DEAE-cellulose column (eluting with a linear gradient of 0 to 0.2 M NaCl in 25 mM Tris-HCl, pH 8.5).

5. Adsorption on Cibachrome Blue-Agarose or hydroxylapatite and elution with 1.5 M KCl or 0.2 M phosphate solution respectively (optional).

6. Molecular sizing on a Sephadex® G-75 column.

7. Cation exchange chromatography on CM-cellulose in 25 mM ammonium acetate at pH 5.0, developed with an ammonium acetate gradient (to 0.2 M ammonium acetate).

The above process yields material of >95% purity.

The material can also be purified by size exclusion chromatography, reverse phase (RP-8) high pressure liquid chromatography or affinity chromatography on immobilized antiinterferon antibodies.

Alternatively the material from step 4 above can be loaded on a monoclonal antibody column, prepared as described by Milstein, Scientific American *243*, 66 (1980), and eluted with 0.2 M acetic acid, 0.1% Triton and 0.15 M NaCl.

In an alternative, preferred embodiment, the mature leukocyte interferon-A produced by the procedure described hereinbefore can be purified by the following steps:

1. Frozen cell pellets containing the expressed mature leukocyte interferon-A are broken up manually or by appropriate size reduction equipment. The partially thawed cells are suspended in 4 volumes of buffer A, containing 0.1 M Tris adjusted to pH 7.5—8.0, 10% (w/v) sucrose, 0.2 M NaCl, 5 mM EDTA, 0.1 mM PMSF and 10—100 mM $MgCl_2$. The suspension is held to approximately 4°C.

The suspension is passed through a homogenizer at about 6000 psi followed by a second pass at less than 1000 psi. Effluent from the homogenizer from both passes is cooled in an ice bath.

2. Polyethylene-imine is added slowly to the homogenate to a concentration of about 0.35% and allowed to stand for about 30 minutes. The solids are removed by centrifugation or filtration. This step is temperature controlled or performed sufficiently quickly that the supernatant (filtrate) is kept at less than 10°C. The supernantant (filtrate) is concentrated by ultrafiltration to approximately 1/10 the original volume. Particulate matter or haziness in the retentate may be removed by an appropriate filter such as a microporous membrane.

3. The clarified solution is loaded directly onto a monoclonal antibody column at a flux of 5—8 cm/hr. (e.g. 25—40 ml/hr. on 2.6 cm diameter column). After loading the column is washed with approximately 10 column volumes of 25 mM Tris-HCl, pH 7.5—8.5, including NaCl (0.5 M) and surfactant such as Triton X-100 (0.2%) or equivalent. Following the wash the column is rinsed with about 10 column volumes of solution containing 0.15 M NaCl and surfactant such as Triton X-100 (0.1%) or equivalent. The column is eluted with 0.2 M acetic acid containing surfactant such as Triton X-100 (0.1%) or equivalent. The protein peak from the monoclonal antibody column (as determined by UV absorbence or other convenient assay) is pooled and the pH adjusted to approximately 4.5 with 1 N NaOH or 1.0 M Tris base.

4. The pooled interferon peak is loaded onto a cationic exchanger such as Whatman CM 52 cellulose or equivalent which has been equilibrated with a suitable buffer such as ammonium acetate pH 4.5 (50 mM). After loading, the column is washed with equilibrating buffer until the UV absorbence of the effluent has reached a plateau so that little additional protein is eluting from the column. The column is then eluted with 25 mM ammonium acetate/0.12 M sodium chloride or a combination which optimizes recovery of interferon and affords a lyophilized cake having satisfactory appearance and solubility properties.

The monoclonal antibodies employed in the preferred embodiment described above can be prepared by the procedures described by Staehelin et al., Proc. Natl. Acad. Sci. U.S.A. *78*, 1848-52 (1981). Monoclonal antibodies are purified and covalently linked to Affigel-10 as described below:

Preparation and purification of monoclonal antibodies from ascitic fluid

Five female Balb/c mice were each inoculated with 5 to $10\times10^6$ hybridoma cells from mid-log growth phase. About $5\times10^6$ viable cells obtained from the mouse producing fluid were inoculated intraperitoneally into each of 10 or more mice. The ascitic fluid was collected repeatedly (2 to 4 times) from each mouse. Up to three transfers and collections may be performed from one group of mice to the next. Ascitic fluid from mice at each transfer was pooled.

Cells and debris were removed from the ascitic fluid by low speed centrifugation (500—1000×g) and 15 minutes. The centrifugation was performed for 90 minutes at 18,000 rpm. The supernatant was frozen and stored at −20°C. After thawing, additional fibrin and particulate material were removed by centrifugation at 35,000 rpm for 90 minutes. Batches of ascitic fluid from each transfer were tested for specific antibody activity by a solid phase antibody-binding assay (Staehelin et al., supra) and pooled if found satisfactory.

Concentration of protein in the pooled solutions was estimated by the approximation that 1 mg of protein yields an absorbance of 1.2 at 280 nm in a cuvette with a path length of 1.0 cm. Ascites fluids with high levels of antibody contain 30 and 35 mg protein/ml. This is equivalent to 4—7 mg of specific antibody/ml. The fluid was diluted with PBS (0.01 M sodium phosphate, pH 7.3, 0.15 M NaCl) to a protein concentration of 10 to 12 mg/ml.

To each 100 ml of diluted solution, 90 ml of room temperature saturated ammonium sulfate solution was added slowly with vigorous stirring at 0°C. The suspension was kept in ice for 40 to 60 minutes, then centrifuged for 15 minutes at 10,000 rpm at 4°C. The supernantant was decanted and drained well. The protein pellets were dissolved in 0.02 M Tris. HCl (pH 7.9)/0.04 M NaCl (Buffer I). The protein solution was dialyzed for 16 to 18 hours at room temperature against 100 volumes of Buffer I with at least one change of the buffer. The dialyzed solution was centrifuged at 15,000 rpm for 10 minutes to remove undissolved material. About 30—35% of the original amount of total protein in the ascitic fluid was recovered as estimated by absorption at 280 nm.

The solution containing 30—40 mg of protein per ml was then applied to a column of DEAE-cellulose equilibrated with Buffer I. A column bed volume of at least 100 ml was used for each gram of protein applied. The antibody was eluted from the column with a linear NaCl gradient contraining 0.02 M Tris.HCl, pH 7.9, from 0.04 M to 0.5 M NaCl. Pooled peak fractions eluting between 0.06 and 0.1 M NaCl were concentrated by precipitation with an equal volume of room temperature saturated ammonium sulfate and centrifugation. The protein pellets were dissolved in 0.2 M $NaHCO_3$ (pH~8.0)/0.3 M NaCl (Buffer II) followed

by dialysis against three changes of the same buffer at room temperature. The dialyzed solutions were centrifuged at 20,000×g for 15 minutes to remove any insoluble material. Protein concentration was adjusted to 20 to 25 mg/ml with Buffer II.

Preparation of immunoadsorbants

Affigel-10 (BioRad Laboratories, Richmond, California) was washed on a sintered glass filter three times with ice-cold isopropanol followed by three washed with ice-cold distilled water. The gel slurry (~50% in cold water) was transferred to plastic tubes and sedimented by a brief centrifugation. The supernatant was aspirated. The packed gel was mixed with an equal volume of purified antibody solutions and rotated end-over-end at 4°C for 5 hours. After reaction, the gel was centrifuged, then washed twice with Buffer III (0.1 M $NaHCO_3$/0.15 M NaCl) to remove uncoupled antibody. Protein determination of the combined washes revealed that more than 90% of antibody was coupled to the gel.

To block unreacted sites, the gel was mixed with an equal volume of 0.1 M ethanolamine. HCl (pH 8) and rotated end-over-end at room temperature for 60 minutes. The gel slurry was washed free of reactants with PBS and stored in PBS in the presence of 0.02% (w/v) sodium azide at 4°C.

K. Parenteral administration

LeIF-A may be parenterally administered to subjects requiring antitumor or antiviral treatment, and to those exhibiting immunosuppressive conditions. Dosage and dose rate may parallel that currently in use in clinical investigations of human derived materials, e.g., about (1—$10\times10^6$ units daily, and in the case of materials of purity greater than 1%, likely up to, e.g., $5\times10^7$ units daily.

As one example of an appropriate dosage form for essentially homogeneous bacterial LeIF-A in parenteral form, 3 mg LeIF-A of specific activity of, say, $2\times10^8$ units/mg may be dissolved in 25 ml of 5 N human serum albumin, the solution is passed through a bacteriological filter and the filtered solution aseptically subdivided into 100 vials, each containing $6\times10^6$ units pure interferon suitable for parenteral administration. The vials are preferably stored in the cold (−20°C) prior to use.

LeIF-A can be formulated according to known methods to prepare pharmaceutically useful compositions, whereby the polypeptide hereof is combined in admixture with a pharmaceutically acceptable carrier vehicle. Suitable vehicles and their formulation are described in Remington's Pharmaceutical Sciences by E. W. Martin, which is hereby incorporated by reference. Such compositions will contain an effective amount of the interferon protein hereof together with a suitable amount of vehicle in order to prepare pharmaceutically acceptable compositions suitable for effective administration to the host. One preferred mode of administration is parenteral.

**Claims**

1. Mature human leukocyte interferon A (LeIF A) characterized by the amino acid sequence Cys Asp Leu Pro Gln Thr His Ser Leu Gly Ser Arg Arg Thr Leu Met Leu Leu Ala Gln Met Arg Lys Ile Ser Leu Phe Ser Cys Leu Lys Asp Arg His Asp Phe Gly Phe Pro Gln Glu Glu Phe Gly Asn Gln Phe Gln Lys Ala Glu Thr Ile Pro Val Leu His Glu Met Ile Gln Gln Ile Phe Asn Leu Phe Ser Thr Lys Asp Ser Ser Ala Ala Trp Asp Glu Thr Leu Leu Asp Lys Phe Tyr Thr Glu Leu Tyr Gln Gln Leu Asn Asp Leu Glu Ala Cys Val Ile Gln Gly Val Gly Val Thr Glu Thr Pro Leu Met Lys Glu Asp Ser Ile Leu Ala Val Arg Lys Tyr Phe Gln Arg Ile Thr Leu Tyr Leu Lys Glu Lys Lys Tyr Ser Pro Cys Ala Trp Glu Val Val Arg Ala Glu Ile Met Arg Ser Phe Ser Leu Ser Thr Asn Leu Gln Glu Ser Leu Arg Ser Lys Glu.

2. A mature human leukocyte interferon characterized by an amino acid sequence as claimed in claim 1 with an additional methionine residue at the amine terminal.

3. A DNA sequence coding for a human leukocyte interferon as claimed in any one of claims 1 or 2.

4. A DNA sequence according to claim 3 comprised in the sequence of Figure 3.

5. The plasmid pLeIF A trp 25.

6. A strain of E. coli transformed with pLeIF A trp 25.

7. E. coli K-12 strain 294 transformed with pLeIF A trp 25.

8. A pharmaceutical composition containing a therapeutically effective amount of a mature human leukocyte interferon-A as claimed in any one of claims 1 or 2 and a carrier material suitable for pharmaceutical administration.

9. A pharmaceutical composition as claimed in claim 8 for parenteral administration.

10. The use of the mature human leukocyte interferon-A claimed in any one of claims 1 or 2 for the preparation of pharmaceutical compositions.

11. A process for preparing a mature human leukocyte interferon-A claimed in any one of claims 1 or 2 which process comprises causing a culture of E. coli, transformed with pLeIF A trp 25, to grow up and express said polypeptide and recoverying said polypeptide.

**Patentansprüche**

1. Reifes menschliches Leukocyteninterferon A (LeIF A) gekennzeichnet durch eine Aminosäuresequenz Cys Asp Leu Pro Gln Thr His Ser Leu Gly Ser Arg Arg Thr Leu Met Leu Leu Ala Gln Met

Arg Lys Ile Ser Leu Phe Ser Cys Leu Lys Asp Arg His Asp Phe Gly Phe Pro Gln Glu Glu Phe Gly Asn Gln Phe Gln Lys Ala Glu Thr Ile Pro Val Leu His Glu Met Ile Gln Gln Ile Phe Asn Leu Phe Ser Thr Lys Asp Ser Ser Ala Ala Trp Asp Glu Thr Leu Leu Asp Lys Phe Tyr Thr Glu Leu Tyr Gln Gln Leu Asp Asp Leu Glu Als Cys Val Ile Gln Gly Val Gly Val Thr Glu Thr Pro Leu Met Lys Glu Asp Ser Ile Leu Ala Val Arg Lys Tyr Phe Gln Arg Ile Thr Leu Tyr Leu Lys Glu Lys Lys Tyr Ser Pro Cys Ala Trp Glu Val Val Arg Ala Glu Ile Met Arg Ser Phe Ser Leu Ser Thr Asn Leu Gln Glu Ser Leu Arg Ser Lys Glu.

2. Ein reifes menschliches Leukozyteninterferon gekennzeichnet durch eine Aminosäuresequenz gemäss Anspruch 1 mit einem zusätzlichen Methioninrest am Aminoende.

3. Eine DNA-Sequenz, die für ein menschliches Leukozytinterferon gemäss einem der Ansprüche 1 oder 2 kodiert.

4. Eine DNA-Sequenz gemäss Anspruch 3, die in der Sequenz von Fig. 3 enthalten ist.

5. Das Plasmid pLeIF A trp 25.

6. Ein E. coli-Stamm, der mit pLeIF A trp 25 transformiert ist.

7. E. coli K-12 Stamm 294, transformiert mit pLeIF A trp 25.

8. Eine pharmazeutische Zusammensetzung enthaltend eine therapeutisch wirksame Menge eines reifen menschlichen Leukozyteninterferons A gemäss einem der Ansprüche 1 oder 2 und ein für pharmazeutische Verabreichung geeignetes Trägermaterial.

9. Eine pharmazeutische Zusammensetzung gemäss Anspruch 8 für parenterale Verabreichung.

10. Die Verwendung von reifem menschlichem Leukozyteninterferon A gemäss einem der Ansprüche 1 oder 2 zur Herstellung von pharmazeutischen Zusammensetzungen.

11. Ein Verfahren zur Herstellung von reifem menschlichem Leukozyteninterferon A gemäss einem der Ansprüche 1 oder 2, dadurch gekennzeichnet, dass man eine Kultur von E. coli, transformiert mit pLeIF A trp 25, aufwachsen und besagtes Polypeptid exprimieren lässt und besagtes Polypeptid gewinnt.

**Revendications**

1. Leucocyte interferon A (LeIF A) humain mûr, caractérisé par sa séquence aminoacide Cys Asp Leu Pro Gln Thr His Ser Leu Gly Ser Arg Arg Thr Leu Met Leu Leu Ala Gln Met Arg Lys Ile Ser Leu Phe Ser Cys Leu Lys Asp Arg His Asp Phe Gly Phe Pro Gln Glu Glu Phe Gly Asn Gln Phe Gln Lys Ala Glu Thr Ile Pro Val Leu His Glu Met Ile Gln Gln Ile Phe Asn Leu Phe Ser Thr Lys Asp Ser Ser Ala Ala Trp Asp Glu Thr Leu Leu Asp Lys Phe Tyr Thr Glu Leu Tyr Gln Gln Leu Asn Asp Leu Glu Ala Cys Val Ile Gln Gly Val Gly Val Thr Glu Thr Pro Leu Met Lys Glu Asp Ser Ile Leu Ala Val Arg Lys Tyr Phe Gln Arg Ile Thr Leu Tyr Leu Lys Glu Lys Lys Tyr Ser Pro Cys Ala Trp Glu Val Val Arg Ala Glu Ile Met Arg Ser Phe Ser Leu Ser Thr Asn Leu Gln Glu Ser Leu Arg Ser Lys Glu.

2. Leucocyte interféron humain mûr, caractérisé par une séquence aminoacide selon la revendication 1 avec un groupe méthionine additionnel au groupe amino terminal.

3. Une séquence DNA codant pour un leucocyte interféron humain selon l'une quelconque des revendications 1 ou 2.

4. Une séquence DNA selon la revendication 3 comprise dans la séquence de la Figure 3.

5. Le plasmide pLeIF A trp 25.

6. Une souche d'E. coli transformée par pLeIF A trp 25.

7. E. coli K-12 souche 294 transformé par pLeIF A trp 25.

8. Composition pharmaceuticque contenant une quantité thérapeutique de leucocyte interféron-A humain mûr revendiqué dans l'une quelconque des revendications 1 ou 2, ainsi qu'un véhicule approprié pour l'administration pharmaceutique.

9. Composition pharmaceutique selon la revendication 8 pour l'administration parentérale.

10. L'utilisation du leucocyte interféron-A humain mûr revendiqué dans l'une quelconque des revendications 1 ou 2 pour la préparation de compositions pharmaceutiques.

11. Procédé pour la préparation du leucocyte interféron-A humain mûr revendiqué dans l'une quelconque des revendications 1 ou 2, caractérisé par le fait qu'on fait croître une culture d'E. coli, transformé par pLeIF A trp 25, exprime ce polypeptide et recouvre ledit polypeptide.

| | TRYPTIC PEPTIDE (T-1) | TRYPTIC PEPTIDE (T-13) |
|---|---|---|
| Protein | Ala-Glu-Ile-Met-Arg | ---His-Glu-Met-Ile-Gln--- |
| mRNA | 5' GCN $GA^{A}_{G}$ $AU\overset{A}{\underset{U}{C}}$ AUG $^{A}_{C}GN$ | 5' $CA^{C}_{U}$ $GA^{A}_{G}$ AUG $AU\overset{A}{\underset{U}{C}}$ $CA^{A}_{G}$ |
| Complementary DNA primers | 3' CTT $TA\overset{A}{\underset{T}{G}}$ TAC GC (T-1A) | 3' GTA CTT TAC TA (T-13A) |
| | --C------------ (T-1B) | --G------------ (T-13B) |
| | -------------T- (T-1C) | -------C-------- (T-13C) |
| | --C----------T- (T-1D) | --G---C-------- (T-13D) |

FIG. 1.

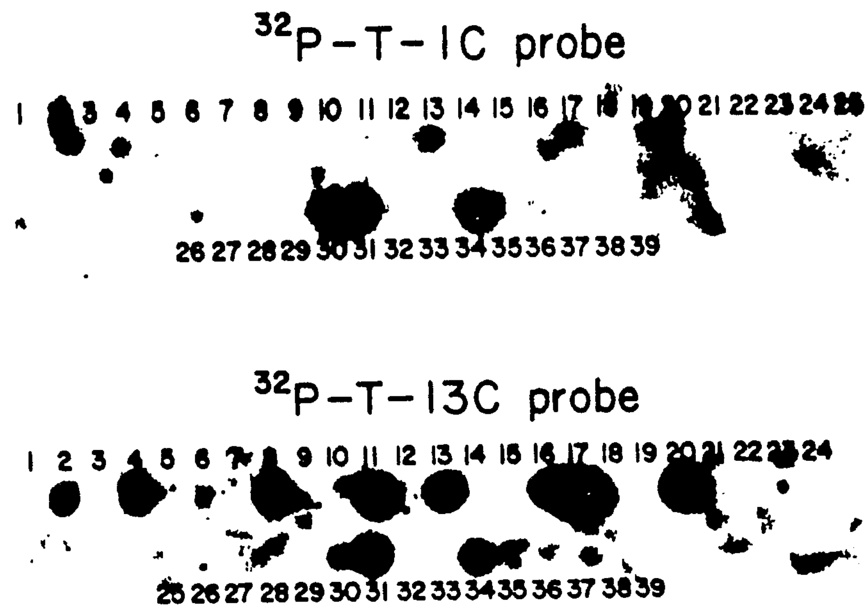


FIG. 2.

-50 . -40 . -20 . +1 . 20 . 40

LeIF A TGAGCCTAAACCTTAGGCTCACCCATTTCAACCAGTCTAGCAGCATCTGCAACATCTACAATGGCCTTGACCTTTGCTTTACTGGTGGCCCTCCTGGTGC

. 60 . 80 . 100 . 120 . 140

LeIF A TCAGCTGCAAGTCAAGCTGCTCTGTGGGCTGTGATCTGCCTCAAACCCACAGCCTGGGTAGCAGGAGGACCTTGATGCTCCTGGCACAGATCAGGAAAAT

. 160 . 180 . 200 . 220 . 240

LeIF A CTCTCTTTTCTCCTGCTTGAAGGACAGACATGACTTTGGATTTCCC CAGGAGGAGTTT GGCAACCAGTTCCAAAAGGCTGAAACCATCCCTGTCCT

. 260 . 280 . 300 . 320 . 340

LeIF A CCATGAGATGATCCAGCAGATCTTCAATCTCTTCAGCACAAAGGACTCATCTGCTGCTTGGGATGAGACCCTCCTAGACAAATTCTACACTGAACTCTAC

. 360 . 380 . 400 . 420 . 440

LeIF A CAGCAGCTGAATGACCTGGAAGCCTGTGTGATACAGGGGGTGGGGGTGACAGAGACTCCCCTGATGAAGGAGGACTCCATTCTGGCTGTGAGGAAATACT

. 460 . 480 . 500 . 520 . 540

LeIF A TCCAAAGAATCACTCTCTATCTGAAAGAGAAGAAATACAGCCCTTGTGCCTGGGAGGTTGTCAGAGCAGAAATCATGAGATCTTTTTCTTTGTCAACAAA

. 560 . 580 . 600 . 620 . 640

LeIF A CTTGCAAGAAAGTTTAAGAAGTAAGGAATGAAAACTGGTTCAACATGGAAATGATTTTCATTGATTCGTATGCCAGCTCACCTTTTTATGATCTGCCATT

. 660 . 680 . 700 . 720 . 740

LeIF A TCAAAGACTCATGTTTCTGCTATGACCATGACACGATTTAAATCTTTTCAAATGTTTTTAGGAGTATTAATCAACATTGTATTCAGCTCTTAAGGCACTA

. 760 . 780 . 800 . 820 . 840

LeIF A GTCCCTTACAGAGGACCATGCTGACTGATCCATTATCTATTTAAATATTTTTAAAATATTATTTATTTAACTATTTATAAAACAACTTATTTTTGTTCAT

. 860 . 880 . 900 . 920 . 940

LeIF A ATTACGTCATGTGCACCTTTGCACAGTGGTTAATGTAATAAAATATGTTCTTTGTATTTGGT-poly(A)

```
       S1              S10                S20  S23           10                  20                  30                  40
LeIF A M A L T F A L L V A L L V L S C K S S C S V G C D L P Q T H S L G S R R T L M L L A Q M R K I S L F S C L K D R H D F G F P Q

                         50                  60                  70                  80                  90                  100
      -E E F - G N Q F Q K A E T I P V L H E M I Q Q I F N L F S T K D S S A A W D E T L L D K F Y T E L Y Q Q L N D L E A C V I Q G

                  110                 120                 130                 140                 150                 160     166
       V G V T E T P L M K E D S I L A V R K Y F Q R I T L Y L K E K K Y S P C A W E V V R A E I M R S F S L S T N L Q E S L R S K E
```

FIG 4.

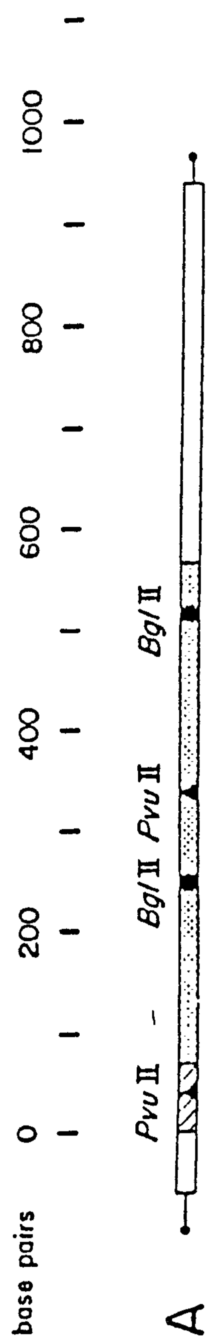


A

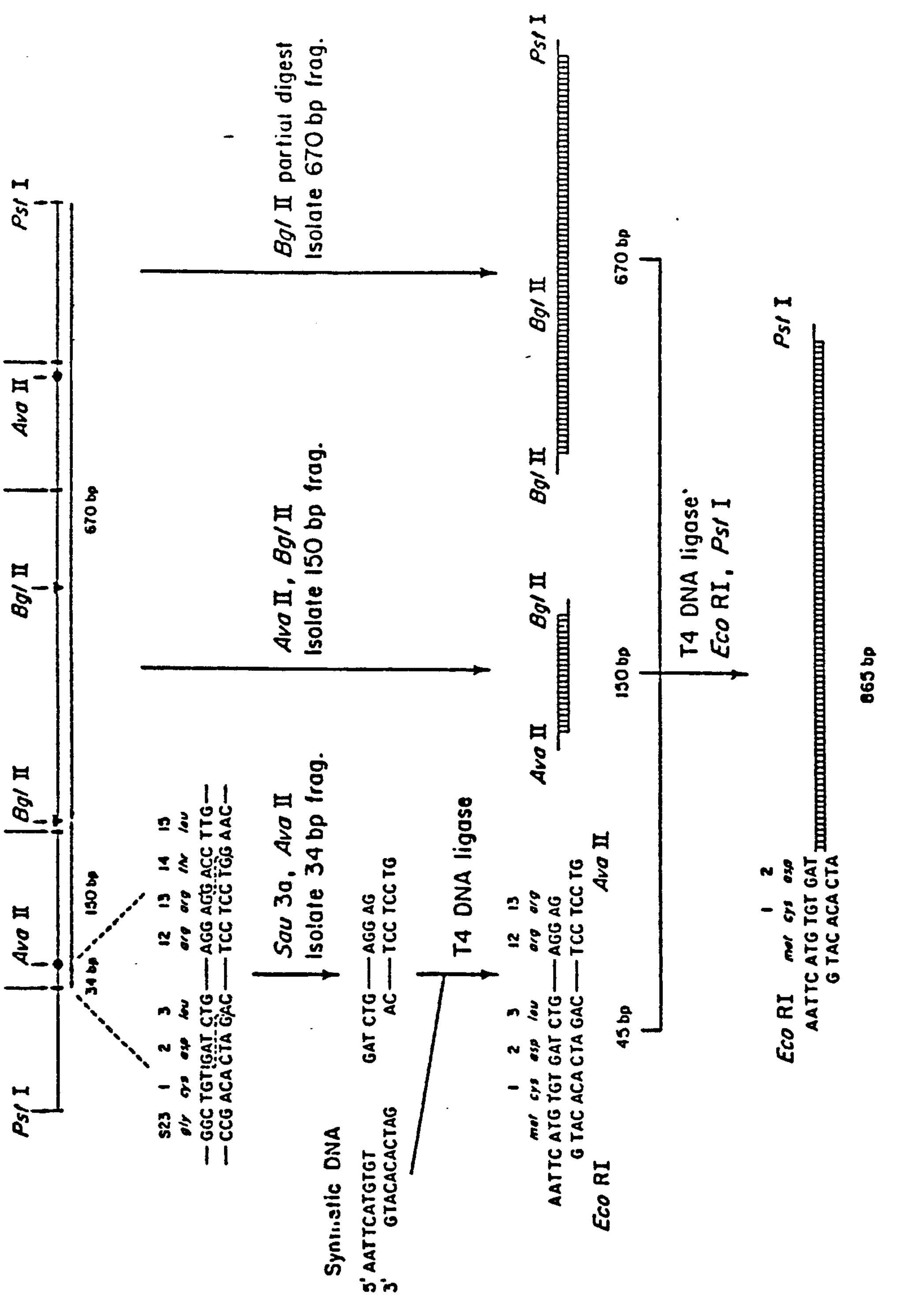

Pst I
Sau 3a
Ava II
Bgl II
34 bp
150 bp
670 bp
S23 1 2 3 12 13 14 15
gly cys asp leu arg arg thr leu
—GGC TGT GAT CTG——AGG AGG ACC TTG—
—CCG ACA CTA GAC——TCC TCC TGG AAC—
Sau 3a, Ava II
Isolate 34 bp frag.
Ava II, Bgl II
Isolate 150 bp frag.
Bgl II partial digest
Isolate 670 bp frag.
Synthetic DNA
5' AATTCATGTGT
3' GTACACACTAG
GAT CTG——AGG AG
AC——TCC TCC TG
T4 DNA ligase
1 2 3 12 13
met cys asp leu arg arg
AATTC ATG TGT GAT CTG——AGG AG
G TAC ACA CTA GAC——TCC TCC TG
Eco RI
45 bp
T4 DNA ligase'
Eco RI, Pst I
1 2
met cys asp
AATTC ATG TGT GAT
G TAC ACA CTA
865 bp


LeIF A

FIG. 6